# FASCICULE DE BREVET EUROPEEN

(11) **EP 1 066 263 B1**
(45) Date de publication et mention de la délivrance du brevet: **26.05.2004**
(21) Numéro de dépôt: 00900644.6
(22) Date de dépôt: 21.01.2000
(51) Int. Cl.: C07D 233/56, A61K 7/13, C07C 233/24

(54) **NOUVEAUX 2-ACYLAMINOPHENOLS CATIONIQUES, LEUR UTILISATION A TITRE DE COUPLEUR POUR LA TEINTURE D'OXYDATION, COMPOSITIONS LES COMPRENANT, ET PROCEDES DE TEINTURE**
KATIONISCHE ACYLAMINOPHENOLE, DEREN VERWENDUNG ALS KUPPLER ZUM OXIDATIVEN FÄRBEN KERATINISCHER FASERN, FÄRBEMITTEL UND FÄRBEVERFAHREN
NOVEL CATIONIC 2-ACYLAMINOPHENOLS, THEIR USE AS COUPLER FOR OXIDATION DYEING, COMPOSITIONS CONTAINING THEM, AND DYEING METHODS

(30) Priorité: 21.01.1999 FR 9900746
(43) Date de publication de la demande: 10.01.2001
(73) Titulaire: L'OREAL, 75008 Paris (FR)
(72) Inventeur: VIDAL, Laurent, F-75013 Paris (FR); SAUNIER, Jean-Baptiste, F-75014 Paris (FR)
(74) Mandataire: Miszputen, Laurent
(86) Numéro de dépôt international: PCT/FR2000/000143
(87) Numéro de publication internationale: WO 2000/043368

(56) Documents cités:
- EP-A- 0 579 204
- WO-A-99/48875
- DE-B- 1 135 589
- FR-A- 2 140 149
- FR-A- 2 233 984

## Description

L'invention a pour objet de nouveaux 2-acylaminophénols cationiques de formule (I) comportant au moins un groupement cationique, leur utilisation à titre de coupleur pour la teinture d'oxydation des fibres kératiniques et en particulier des fibres kératiniques humaines telles que les cheveux, les compositions de teinture d'oxydation les contenant en association avec au moins une base d'oxydation, et les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour obtenir des nuances rouges, on utilise généralement, seul ou en mélange avec d'autres bases, et en association avec des coupleurs appropriés, du 4-aminophénol, et pour obtenir des nuances bleues, on fait habituellement appel à des paraphénylènediamines. L'utilisation de coupleurs dérivés de métaphénylènediamines, en association avec des dérivés de paraphénylènediamines, conduit habituellement à des nuances bleues de solidité généralement médiocre.

Il a déjà été proposé, notamment dans le brevet FR-A-1 596 879, d'utiliser pour la teinture d'oxydation des fibres kératiniques, des dérivés phénoliques substitués en position 2 par un radical uréinyle ou thiouréinyle, en association avec des dérivés de paraphénylènediamines, afin d'obtenir des nuances proches de celles obtenues avec les coupleurs dérivés de métaphénylènediamines . Toutefois, les compositions tinctoriales contenant les composés cités dans ce brevet conduisent généralement sur cheveux à des couleurs trop sélectives et manquant d'intensité.

Par ailleurs, il a déjà été proposé, notamment dans le brevet BE 816 674, d'utiliser pour la teinture de fibres kératiniques, en association avec des dérivés de paraphénylènediamines, des dérivés phénoliques substitués en position 2 par un radical acétyle ou ureïque et en position 5 par un atome d'halogène, afin d'obtenir des teintures allant du vert au bleu-vert. Les ténacités à la lumière des nuances obtenues sur cheveux en mettant en oeuvre ces compositions sont généralement meilleures que celles obtenues avec des compositions tinctoriales contenant un ou plusieurs métaphénylènediamines à titre de coupleurs. Cependant, les ténacités aux intempéries et aux lavages, ainsi que les intensités des colorations obtenues sont encore trop faibles et constituent en ces points des inconvénients majeurs pour l'homme de l'art.

En outre, il a déjà été proposé, notamment dans la demande de brevet EP 0 579 204, d'utiliser pour la teinture de fibres kératiniques, des dérivés phénoliques non cationiques substitués en position 2 par un radical acylamino, carbamoyle ou uréyles, et en position 5 par un radical alkyle en C₁-C₄, en association avec des dérivés de paraphénylènediamine. Toutefois, l'utilisation des dérivés phénoliques cités dans cette demande de brevet européen ne permet pas d'obtenir une riche palette de couleurs, et de plus les nuances bleues généralement obtenues ne donnent pas entièrement satisfaction en ce qui concerne leur résistance aux lavages et à l'action de la lumière.

### Le document WO 99148875 décrit le composé 1,4-bis-{3-[2,4-dihydroxyphenylcarbamoyl)-méthyl]-3H-imidazol-1-ium} butane.

Or la demanderesse vient maintenant de découvrir , de façon totalement inattendue et surprenante, que nouveaux 2-acylaminophénols de formule (I) définie ci-après comportant au moins un groupement cationique de formule (II) définie ci-après, non seulement conviennent pour une utilisation comme coupleur, mais en outre qu'ils permettent d'obtenir des compositions tinctoriales conduisant à des colorations puissantes, dans une large palette de couleurs, et présentant d'excellentes propriétés de résistance aux différents traitements que peuvent subir les fibres kératiniques.

Ces découvertes sont à la base de la présente invention.

L'invention a donc pour premier objet de nouveaux 2-acylaminophénols cationiques de formule (I) suivante et leurs sels d'addition avec un acide : 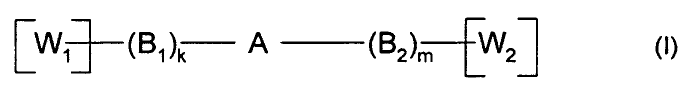 dans laquelle :
- le groupement A représente un groupement imidazolidinium, N-alkyl(C₁-C₄)pyridinium, N-(2-hydroxyéthyl)pyridinium, pyridinium, dialkyl(C₁-C₄)ammonium, 1,4-dimétylpipérazinium-1-yl;
- B₁ et B₂, indépendamment l'un de l'autre, représentent un bras -(CH2)-, -(CH2)-(CH2)-, ou -(CH2)-(CH2)-(CH2)-;
- k et m sont des nombres entiers, qui peuvent prendre, indépendamment les uns des autres, la valeur 0 ou 1;
- W₁ et W₂ représentent, indépendamment l'un de l'autre, un groupement W représenté par la formule (II) suivante : 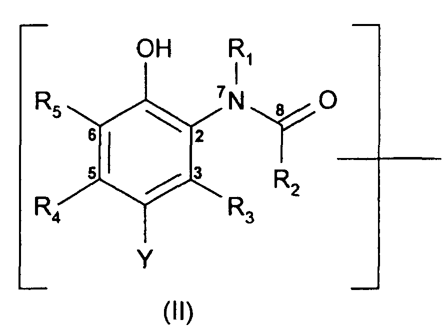 formule (II) dans laquelle :
   - R₁ représente un atome d'hydrogène ; un radical méthyle, éthyle, isopropyle;
   - R₂ représente un radical choisi parmi un radical méthyle, éthyle, propyle, isopropyle, butyle, isobutyle, tert-butyle, pentyle, isopentyle, néopentyle, hexyle; cyclopropyle, cyclobutyle, cyclopentyle, cyclopentylméthyle, 3-cyclopentyl-propyle, cyclohexyle, 2-cyclohexyl-éthyle, hydroxyméthyle, méthoxyméthyle, 1,2-dihydroxyéthyle, 2-carboxyéthyle, 2-(méthoxycarbonyl)éthyle, 2-carboxycylopropyle; méthoxy, éthoxy, propoxy, isopropoxy, butoxy, isobutoxy, pentoxy, néopentoxy, hexyloxy, cyclopentyloxy ; cyclohexyloxy, amino, méthylamino, éthylamino, propylamino, isopropylamino, butylamino, cyclohexylamino, diméthylamino ;
   - R₃ représente un atome d'hydrogène ou de chlore ; un radical méthyle, hydroxyméthyle, méthoxyméthyle, 1-hydroxyéthyle, aminométhyle, méthylaminométhyle, hydroxy, méthoxy, amino, méthylamino, ou 2-hydroxyéthylamino ;
   - R₄ représente un atome d'hydrogène ou de chlore ; un radical méthyle, éthyle, hydroxyméthyle, méthoxyméthyle, aminométhyle, méthylaminométhyle, hydroxy, méthoxy, acétoxy, amino, méthylamino ;
   - R₅ représente un atome d'hydrogène, de chlore, ou de brome ; un radical méthyle, méthoxy, ou méthylamino ;
   - Y représente un atome d'hydrogène, de chlore, de fluor, ou de brome ; un groupement méthoxy, éthoxy, propoxy, benzyloxy, phénoxy, -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅ ;
   étant entendu que :
   - le radical B₁ est relié au groupement A par l'un quelconque des atomes du radical A ;
   - lorsque k = 0, alors le groupement A peut être relié au groupement W₁ directement par l'atome d'azote de l'ammonium quaternaire,
   - le radical B₂ est relié au groupement A par l'un quelconque des atomes du radical A ;
   - lorsque m = 0, alors le groupement A peut être relié au groupement W₂ directement par l'atome d'azote de l'ammonium quaternaire,
   - le groupement B₁ de la formule (t) est relié à un atome du groupement W₁ et que cet atome est identifié par un astérisque (*) sur le squelette de W₁ représenté par la formule (III) définie ci-dessous ;
   - le groupement B₂ est relié à un atome du groupement W₂ et que cet atome est identifié par un astérisque (*) sur le squelette de W₂ représenté par la formule (III) définie ci-dessous ;

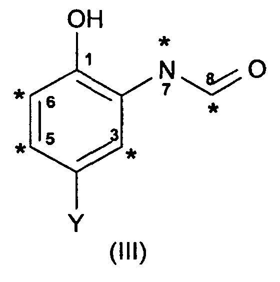

Comme indiqué précédemment, la composition de teinture d'oxydation contenant le ou les composés de formule (I) conforme à l'invention permet d'obtenir des colorations puissantes dans des nuances allant du rouge au bleu et présentant de plus une ténacité remarquable aux différents traitements que peuvent subir les fibres kératiniques. Ces propriétés sont particulièrement remarquables notamment en ce qui concerne la résistance des colorations obtenues vis à vis de l'action de la lumière, des intempéries, des lavages, de l'ondulation permanente et de la transpiration.

De façon plus préférentielle, R₂ représente un radical choisi parmi un radical méthyle, éthyle, propyle, 1,2-dihydroxyéthyle, 2-carboxyéthyle, méthoxy, éthoxy, propoxy, amino, éthylamino, diméthylamino.

De façon encore plus préférentielle, R₂ représente un radical méthyle, méthoxyméthyle, 2-carboxyéthyle, méthoxy, amino, éthylamino.

De façon préférentielle, R₃ représente un atome d'hydrogène ; un radical méthyle, hydroxyméthyle, aminométhyle, hydroxy, méthoxy, amino, méthylamino.

R₅ représente de préférence un atome d'hydrogène, de chlore, ou de brome ; un radical méthyle, méthoxy, ou méthylamino.

De façon préférentielle, Y représente un atome d'hydrogène ou de chlore ; ou un groupement méthoxy, -OCH₂(CO)OH, ou -OCH₂(CO)OCH₃.

Parmi les composés de formule (I) ci-dessus, on peut tout particulièrement citer :
- le chlorure de 1,3-bis-[(2-hydroxy-phénylcarbamoyl)-méthyl]-3H-imidazol-1-ium ;
- le chlorure de 1,3-bis-[(2-hydroxy-4-méthyl-phénylcarbamoyl)-méthyl]-3H-imidazol-1-ium ;
- le chlorure de 1,3-bis-[(2-hydroxy-4-amino-phénylcarbamoyl)-méthyl]-3H-imidazol-1-ium ;
- le chlorure de 1,3-bis-[(2-hydroxy-4-acétylamino-phénylcarbamoyl)-méthyl]-3H-imidazol-1-ium ;
- le chlorure de 1,3-bis-[(2-hydroxy-4-méthoxycarbonylamino-phénylcarbamoyl)méthyl]-3H-imidazol-1-ium ;
- le chlorure de 1,3-bis-[(2-hydroxy-5-chloro-phénylcarbamoyl)-méthyl]-3H-imidazol-1-ium ;
- le chlorure de 1,3-bis-[(2-hydroxy-4-méthyl-5-chloro-phénylcarbamoyl)-méthyl]-3H-imidazol-1-ium ;
- le chlorure de 1,3-bis-[(2-hydroxy-4-amino-5-chloro-phénylcarbamoyl)-méthyl]-3H-imidazol-1-ium;
- le chlorure de 1,3-bis-[(2-hydroxy-4-acétylamino-5-chloro-phénylcarbamoyl)-méthyl]-3H-imidazof-1-ium ;
- le chlorure de 1,3-bis-[(2-hydroxy-5-méthoxy-phénylcarbamoyl)-méthyl]-3H-imidazol-1-ium ;
- le chlorure de 1,3-bis-[(2-hydroxy-4-méthyl-5-méthoxy-phénylcarbamoyl)-méthyl]-3H-imidazol-1-ium ;
- le chlorure de 1,3-bis-[(2-hydroxy-4-amino-5-méthoxy-phénylcarbamoyl)-méthyl]-3H-imidazol-1-ium ;
- le chlorure de 1,3-bis-[(2-hydroxy-4-acétylamino-5-méthoxy-phénylcarbamoyl)-méthyl]-3H-imidazol-1-ium ;
- le chlorure de 1,3-bis-[(2-hydroxy-6-amino-phénylcarbamoyl)-méthyl]-3H-imidazol-1-ium ;
- le chlorure de 1,3-bis-[(2-hydroxy-4,6-diamino-phénylcarbamoyl)-méthyl]-3H-imidazol-1-ium ;
- le chlorure de 1,3-bis-[(2-hydroxy-4-acétylamino-6-amino-phénylcarbamoyl)-méthyl]-3H-imidazol-1-ium ;
- le chlorure de 1,3-bis-[(2-hydroxy-3,5-dichloro-4-méthyl-phénylcarbamoyl)-méthyl]-3H-imidazol-1-ium ;
- le chlorure de 1,3-bis-[(2-hydroxy-3,5-dichloro-4-amino-phénylcarbamoyl)-méthyl]-3H-imidazol-1-ium ;
- le chlorure de 1,3-bis-[(2-hydroxy-3,5-dichloro-4-acétylamino-phénylcarbamoyl)-méthyl]-3H-imidazol-1-ium ;
- le chlorure de 3-[(2-hydroxy-4-amino-phénylcarbamoyl)-méthyl]-1-[(2-hydroxy-4-méthyl-phénylcarbamoyl)-méthyl]-3H-imidazol-1-ium ;
- le chlorure de 3-[(2-hydroxy-3,5-dichloro-4-méthyl-phénylcarbamoyl)-méthyl]-1-[(2-hydroxy-4-méthyl-phénylcarbamoyl)-méthyl]-3H-imidazol-1-ium ;
- le chlorure de 1,4-bis-[(2-hydroxy-phénylcarbamoyl)-méthyl]-1-méthyl-pipérazin-1-ium ;
- le chlorure de 1,4-bis-[(2-hydroxy-4-méthyl-phénylcarbamoyl)-méthyl]-1-méthyl-pipérazin-1-ium ;
- le chlorure de 1,4-bis-[(2-hydroxy-4-amino-phénylcarbamoyl)-méthyl]-1-méthyl-pipérazin-1-ium ;
- le chlorure de 1,4-bis-[(2-hydroxy-4-acétylamino-phénylcarbamoyl)-méthyl]-1-méthyl-pipérazin-1-ium ;
- le chlorure de 1,4-bis-[(2-hydroxy-5-chloro-phénylcarbamoyl)-méthyl]-1-méthyl-pipérazin-1-ium ;
- le chlorure de 1,4-bis-[(2-hydroxy-4-méthyl-5-chloro-phénylcarbamoyl)-méthyl]-1-méthyl-pipérazin-1-ium ;
- le chlorure de 1,4-bis-[(2-hydroxy-4-amino-5-chloro-phénylcarbamoyl)-méthyl]-1-méthyl-pipérazin-1-ium;
- le chlorure de 1,4-bis-[(2-hydroxy-4-acétylamino-5-chloro-phénylcarbamoyl)-méthyl]-1-méthyl-pipérazin-1-ium ;
- le chlorure de 1,4-bis-[(2-hydroxy-5-méthoxy-phénylcarbamoyl)-méthyl]-1-méthyl-pipérazin-1-ium ;
- le chlorure de 1,4-bis-[(2-hydroxy-4-méthyl-5-méthoxy-phénylcarbamoyl)-méthyl]-1-méthyl-pipérazin-1-ium ;
- le chlorure de 1,4-bis-[(2-hydroxy-4-amino-5-méthoxy-phénylcarbamoyl)-méthyl]-1-méthyl-pipérazin-1-ium ;
- le chlorure de 1,4-bis-[(2-hydroxy-4-acétylamino-5-méthoxy-phénylcarbamoyl)-méthyl]-1-méthyl-pipérazin-1-ium ;
- le chlorure de 1,4-bis-[(2-hydroxy-6-amino-phénylcarbamoyl)-méthyl]-1-méthyl-pipérazin-1-ium ;
- le chlorure de 1,4-bis-[(2-hydroxy-4,6-diamino-phénylcarbamoyl)-méthyl]-1-méthyl-pipérazin-1-ium ;
- le chlorure de 1,4-bis-[(2-hydroxy-4-acétylamino-6-amino-phénylcarbamoyl)-méthyl]-1-méthyl-pipérazin-1-ium ;
- le chlorure de 1,4-bis-[(2-hydroxy-3,5-dichloro-4-méthyl-phénylcarbamoyl)-méthyl]-1-méthyl-pipérazin-1-ium ;
- le chlorure de 1,4-bis-[(2-hydroxy-3,5-dichloro-4-amino-phénylcarbamoyl)-méthyl]-1-méthyl-pipérazin-1-ium ;
- le chlorure de 1,4-bis-[(2-hydroxy-3,5-dichloro-4-acétylamino-phénylcarbamoyl)-méthyl]-1-méthyl-pipérazin-1-ium ;
et leurs sels d'addition avec un acide.

Les composés de formule (I) conformes à l'invention peuvent être préparés selon des méthodes bien connues de l'état de la technique et décrites par exemple dans les demandes de brevet ou brevets FR-A-1 596 879 ; BE 816 674 ; EP 0 579 204 ; DE 2 846 931 ; JP-54-115 230 ; GB 2 070 000 ; DE 3 027 128 ; EP 0 065 874 ; EP0 115 194 ; EP 0 079 141 ; EP 0 081 321 ; DE 3 246 238 ; EP 0 168 729 ; DE 3 414 051 ; JP-59-059656 ; FR-A-2 575 470; EP 0 193 051 ; JP-63-208562 ; JP-62-173469 ; JP-62-108859 ; JP-62-173469 ; DD253997 ; DE 3 641 825 , JP-63-208562 ; DE 3 621 215 ; JP-01-249739 ; JP-64-002045 ; JP-02-255674 ; JP-01-032261 ; JP-02-255674 ; EP 0 608 896 , WO 94/19316 ; JP-09-169705 ; EP 0 790 240 ; ainsi que dans les documents Res. Discl. (1981), 202, 76-8 ; Synthesis (1982), 940-2 ; Res. Discl. (1983), 235, 352-3 ; Res. Discl. (1984), 247, 554-6 ; Res. Discl. (1985), 251, 134-9 ; Chem. Ind. (Dekker) (1992), 47 (Catal. Org. React.), 147-51 ; et J. Med. Chem: (1998), 41, 4062-79.

Un autre objet de l'invention est l'utilisation des composés de formule (I) conformes à l'invention à titre de coupleur pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) conforme à l'invention et au moins une base d'oxydation.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale conforme à l'invention n'est pas critique. Elles sont de préférence choisies parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénytènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, les compositions tinctoriales renfermant une ou plusieurs paraphénylènediamines et/ou une ou plusieurs bases d'oxydation hétérocycliques sont particulièrement préférées.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 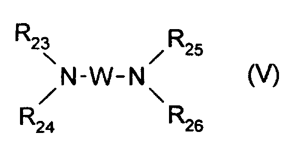 dans laquelle W est un reste propylène substitué ou non substitué par un groupement hydroxyle ou un radical alkyle en C₁-C₆ ; R₂₃, R₂₄, R₂₅ et R₂₆, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. II est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples sui suivent sont destinés à illustrer l'invention.

### EXEMPLE DE PREPARATION : Synthèse du chlorure de 1,3-bis-[(2-hydroxy-4-méthyl-phénylcarbamoyl)-méthyl]-3H-imidazol-1-ium.

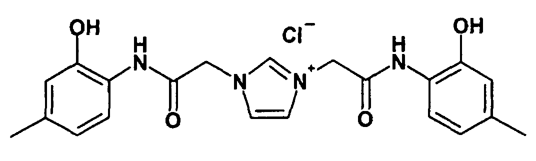

### a) Préparation du de 2-chloro-N-(2-hydroxy-4-méthyl-phényl)-acétamide

A une solution de 83 g de 2-hydroxy-4-méthylaniline (0,674 mole) dans 2,5 litre de tétrahydrofurane, sous agitation et sous atmosphère inerte, ont été ajoutés 95 ml de triéthylamine (0,674 mole), puis après 30 minutes on a ajouté goutte à goutte une solution de 54 ml de chlorure de chloroacétyle (0,678 mole) dans 60 ml de tétrahydrofurane, en maintenant la température à 25-27°C. Le milieu réactionnel a été agité à température ambiante pendant 4 heures, filtré sur verre fritté et les sels minéraux ont été rincés par 500 ml de tétrahydrofurane. Les phases organiques combinées ont été versées sur 3 litres d'eau glacée sous agitation. Le précipité formé a été essoré, lavé par deux fois 600 ml d'eau et une fois au pentane, et séché sous vide jusqu'à poids constant pour donner 99 g de 2-chloro-N-(2-hydroxy-4-méthyl-phényl)-acétamide sous la forme d'une poudre beige, avec un rendement de 74%.

### b) Préparation du chlorure de 1,3-bis-[(2-hydroxy-4-méthyl-phénylcarbamoyl)-méthyl]-3H-imidazol-1-ium

Une solution de 2-chloro-N-(2-hydroxy-4-méthyl-phényl)-acétamide (6 g, 30 mmoles) obtenu ci-dessus à l'étape précédente, et d'imidazole (680 mg, 10 mmoles) dans 40 ml de 1,2-diméthoxy-éthylène a été chauffée au reflux pendant 48 heures. Le précipité formé a été essoré, lavé deux fois au 1,2-diméthoxy-éthylène. Les 2 g de poudre blanche ainsi obtenue ont été mis en suspension dans 100 ml d'eau. Le pH du milieu réactionnel a été porté à 7,0 par adjonction d'une solution de soude (10%, puis 0,1%). Le précipité a été essoré et lavé avec un minimum d'eau, puis agité pendant une heure dans 800 ml d'eau. La suspension a été filtrée. Le filtrat aqueux a été concentré sous vide et séché sous vide jusqu'à poids constant pour donner 0,5 g de chlorure de 1,3-bis-[(2-hydroxy-4-méthyl-phénylcarbamoyl)-méthyl]-3H-imidazol-1-ium sous la forme d'une poudre blanche fondant à 232°C, et la spectroscopie de masse cation : m/z=395 était conforme au produit attendu.

### EXEMPLES DE TEINTURE

### EXEMPLES 1 à 3 DE TEINTURE EN MILIEU ALCALIN

On a préparé les compositions tinctoriales suivantes (teneurs en grammes) :

| **EXEMPLE** | **1** | **2** | **3** |
|---|---|---|---|
| Chlorure de 1,3-bis-[(2-hydroxy-4-méthylphénylcarbamoyl)-méthyl]-3H-imidazol-1-ium (composé de formule (I)) | 1,30 | 1,30 | 1,30 |
| Paraphénylènediamine (base d'oxydation) | 0,324 | - | - |
| Pyrazolo-[1,5-a]-pyrimidine-3,7-diamine, 2HCl (base d'oxydation) | - | 0,666 | - |
| N,N-bis hydroxyéthyl paraphénylènediamine, 2HCl (base d'oxydation) | - | - | 0,882 |
| Support de teinture commun n°1 | (*) | (*) | (*) |
| Eau déminéralisée q.s.p. | 100 g | 100 g | 100 g |
| (*) Support de teinture commun n° 1 : - Alcool benzylique 2,0 g - Polyéthylène glycol à 6 moles d'oxyde d'éthylène 3,0 g - Ethanol à 96° 20,0 g - Alkyl (C₈-C₁₀) polyglucoside en solution aqueuse à 60 % de matière active (M.A.), tamponnée par du citrate d'ammonium, vendu sous la dénomination ORAMIX CG 110 ® par la société SEPPIC 6,0 g - Ammoniaque à 20% de NH₃ 10,0 g - Métabisulfite de sodium à 35% de matière active 0,228 g - Sel pentasodique de l'acide diéthylènetriaminopentacétique 1,1 g | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Châtain clair cendré légèrement bleuté |
| **2** | 10 ± 0,2 | Blond irisé |
| **3** | 10 ± 0,2 | Bleu mat |

## Revendications

1. 2-acylaminophénols cationiques de formule (I) suivante et leurs sels d'addition avec un acide : 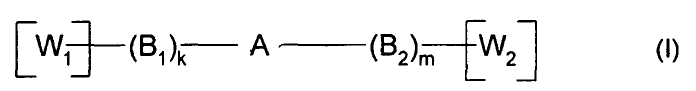 dans laquelle :
- le groupement A représente un groupement imidazolidinium; N-alkyl(C₁-C₄)pyridinium, N-(2-hydroxyéthyl)pyridinium, pyridinium, dialkyl(C₁-C₄)ammonium, 1,4-dimétylpipérazinium-1-yl;
- B₁ et B₂ , indépendamment l'un de l'autre, représentent un bras -(CH2)-, -(CH2)-(CH2)-, ou -(CH2)-(CH2)-(CH2)-;
- k et m sont des nombres entiers, qui peuvent prendre, indépendamment les uns des autres, la valeur 0 ou 1;
- W₁ et W₂ représentent, indépendamment l'un de l'autre, un groupement W représenté par la formule (II) suivante : 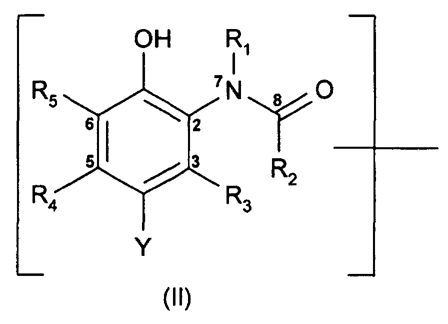 formule (II) dans laquelle :
• R₁ représente un atome d'hydrogène ; un radical méthyle, éthyle, isopropyle ;
• R₂ représente un radical choisi parmi un radical méthyle, éthyle, propyle, isopropyle, butyle, isobutyle, tert-butyle, pentyle, isopentyle, néopentyle, hexyle; cyclopropyle, cyclobutyle, cyclopentyle, cyclopentylméthyle, 3-cyclopentyl-propyle, cyclohexyle, 2-cyclohexyl-éthyle, hydroxyméthyle, méthoxyméthyle, 1,2-dihydroxyéthyle, 2-carboxyéthyle, 2-(méthoxycarbonyl)éthyle, 2-carboxycylopropyle ; méthoxy, éthoxy, propoxy, isopropoxy, butoxy, isobutoxy, pentoxy, néopentoxy, hexyloxy, cyclopentyloxy, cyclohexyloxy ; amino, méthylamino, éthylamino, propylamino, isopropylamino, butylamino, cyclohexylamino, diméthylamino ;
• R₃ représente un atome d'hydrogène ou de chlore ; un radical méthyle, hydroxyméthyle, méthoxyméthyle, 1-hydroxyéthyle, aminométhyle, méthylaminométhyle, hydroxy, méthoxy, amino, méthylamino, ou 2-hydroxyéthylamino ;
• R₄ représente un atome d'hydrogène ou de chlore ; un radical méthyle, éthyle, hydroxyméthyle, méthoxyméthyle, aminométhyle, méthylaminométhyle, hydroxy, méthoxy, acétoxy, amino, méthylamino ;
• R₅ représente un atome d'hydrogène, de chlore, ou de brome ; un radical méthyle, méthoxy, ou méthylamino ;
• Y représente un atome d'hydrogène, de chlore, de fluor, ou de brome ; un groupement méthoxy, éthoxy, propoxy, benzyloxy, phénoxy, -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅ ;
étant entendu que :
- le radical B₁ est relié au groupement A par l'un quelconque des atomes du radical A ;
- lorsque k = 0, alors le groupement A peut être relié au groupement W₁ directement par l'atome d'azote de l'ammonium quaternaire,
- le radical B₂ est relié au groupement A par l'un quelconque des atomes du radical A ;
- lorsque m = 0, alors le groupement A peut être relié au groupement W₂ directement par l'atome d'azote de l'ammonium quaternaire,
- le groupement B₁ de la formule (I) est relié à un atome du groupement W₁ et que cet atome est identifié par un astérisque (*) sur le squelette de W₁ représenté par la formule (III) définie ci-dessous ;
- le groupement B₂ est relié à un atome du groupement W₂ et que cet atome est identifié par un astérisque (*) sur le squelette de W₂ représenté par la formule (III) définie ci-dessous ;

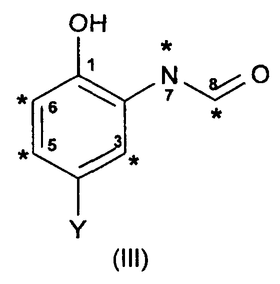

2. Composés selon la revendication 1, **caractérisés par le fait que** R₂ représente un radical choisi parmi un radical méthyle, éthyle, propyle, 1,2-dihydroxyéthyle, 2-carboxyéthyle, méthoxy, éthoxy, propoxy, amino, éthylamino, diméthylamino.

3. Composés selon la revendication 2, **caractérisés par le fait que** R₂ représenté un radical méthyle, méthoxyméthyle, 2-carboxyéthyle, méthoxy, amino, éthylamino.

4. Composés selon la revendication 1, **caractérisés par le fait que** R₃ représente un atome d'hydrogène, un radical méthyle, hydroxyméthyle, aminométhyle, hydroxy, méthoxy, amino, méthylamino.

5. Composés selon la revendication 1, **caractérisés par le fait que** R₅ représente un atome d'hydrogène, de chlore, un radical méthyle, méthoxy, ou méthylamino.

6. Composés selon l'une quelconque des revendications précédentes, **caractérisés par le fait qu'**ils sont choisis parmi :
- le chlorure de 1,3-bis-[(2-hydroxy-phénylcarbamoyl)-méthyl]-3H-imidazol-1-ium ;
- le chlorure de 1,3-bis-[(2-hydroxy-4-méthyl-phénylcarbamoyl)-méthyl]-3H-imidazol-1-ium ;
- le chlorure de 1,3-bis-[(2-hydroxy-4-amino-phénylcarbamoyl)-méthyl]-3H-imidazol-1-ium ;
- le chlorure de 1,3-bis-[(2-hydroxy-4-acétylamino-phénylcarbamoyl)-méthyl]-3H-imidazol-1-ium ;
- le chlorure de 1,3-bis-[(2-hydroxy-4-méthoxycarbonylamino-phénylcarbamoyl)-méthyl]-3H-imidazol-1-ium ;
- le chlorure de 1,3-bis-[(2-hydroxy-5-chloro-phénylcarbamoyl)-méthyl]-3H-imidazol-1-ium ;
- le chlorure de 1,3-bis-[(2-hydroxy-4-méthyl-5-chloro-phénylcarbamoyl)-méthyl]-3H-imidazol-1-ium ;
- le chlorure de 1,3-bis-[(2-hydroxy-4-amino-5-chloro-phénylcarbamoyl)-méthyl]-3H-imidazol-1-ium ;
- le chlorure de 1,3-bis-[(2-hydroxy-4-acétylamino-5-chloro-phénylcarbamoyl)-méthyl]-3H-imidazol-1-ium ;
- le chlorure de 1,3-bis-[(2-hydroxy-5-méthoxy-phénylcarbamoyl)-méthyl]-3H-imidazol-1-ium ;
- le chlorure de 1,3-bis-[(2-hydroxy-4-méthyl-5-méthoxy-phénylcarbamoyl)-méthyl]-3H-imidazol-1-ium ;
- le chlorure de 1,3-bis-[(2-hydroxy-4-amino-5-méthoxy-phénylcarbamoyl)-méthyl]-3H-imidazol-1-ium ;
- le chlorure de 1,3-bis-[(2-hydroxy-4-acétylamino-5-méthoxy-phénylcarbamoyl)-méthyl]-3H-imidazol-1-ium ;
- le chlorure de 1,3-bis-[(2-hydroxy-6-amino-phénylcarbamoyl)-méthyl]-3H-imidazol-1-ium ;
- le chlorure de 1,3-bis-[(2-hydroxy-4,6-diamino-phénylcarbamoyl)-méthyl]-3H-imidazol-1-ium ;
- le chlorure de 1,3-bis-[(2-hydroxy-4-acétylamino-6-amino-phénylcarbamoyl)-méthyl]-3H-imidazol-1-ium ;
- le chlorure de 1,3-bis-[(2-hydroxy-3,5-dichloro-4-méthyl-phénylcarbamoyl)-méthyl]-3H-imidazol-1-ium ;
- le chlorure de 1,3-bis-[(2-hydroxy-3,5-dichloro-4-amino-phénylcarbamoyl)-méthyl]-3H-imidazol-1-ium;
- le chlorure de 1,3-bis-[(2-hydroxy-3,5-dichloro-4-acétylamino-phénylcarbamoyl)-méthyl]-3H-imidazol-1-ium ;
- le chlorure de 3-[(2-hydroxy-4-amino-phénylcarbamoyl)-méthyl]-1-[(2-hydroxy-4-méthyl-phénylcarbamoyl)-méthyl]-3H-imidazol-1-ium ;
- le chlorure de 3-[(2-hydroxy-3,5-dichloro-4-méthyl-phénylcarbamoyl)-méthyl]-1-[(2-hydroxy-4-méthyl-phénylcarbamoyl)-méthyl]-3H-imidazol-1 -ium ;
- le chlorure de 1,4-bis-((2-hydroxy-phénylcarbamoyl)-méthyl]-1-méthyl-pipérazin-1-ium ;
- le chlorure de 1,4-bis-[(2-hydroxy-4-méthyl-phénylcarbamoyl)-méthyl]-1-méthyl-pipérazin-1-ium ;
- le chlorure de 1,4-bis-[(2-hydroxy-4-amino-phénylcarbamoyl)-méthyl]-1-méthyl-pipérazin-1-ium;
- le chlorure de 1,4-bis-[(2-hydroxy-4-acétylamino-phénylcarbamoyl)-méthyl]-1-méthyl-pipérazin-1-ium ;
- le chlorure de 1,4-bis-[(2-hydroxy-5-chloro-phénylcarbamoyl)-méthyl]-1-méthyl-pipérazin-1-ium ;
- le chlorure de 1,4-bis-[(2-hydroxy-4-méthyl-5-chloro-phénylcarbamoyl)-méthyl]-1-méthyl-pipérazin-1-ium ;
- le chlorure de 1,4-bis-[(2-hydroxy-4-amino-5-chloro-phénylcarbamoyl)-méthyl]-1-méthyl-pipérazin-1-ium ;
- le chlorure de 1,4-bis-[(2-hydroxy-4-acétylamino-5-chloro-phénylcarbamoyl)-méthyl]-1-méthyl-pipérazin-1-ium ;
- le chlorure de 1,4-bis-[(2-hydroxy-5-méthoxy-phénylcarbamoyl)-méthyl]-1-méthyl-pipérazin-1-ium ;
- le chlorure de 1,4-bis-[(2-hydroxy-4-méthyl-5-méthoxy-phénylcarbamoyl)-méthyl]-1-méthyl-pipérazin-1-ium ;
- le chlorure de 1,4-bis-[(2-hydroxy-4-amino-5-méthoxy-phénylcarbamoyl)-méthyl]-1-méthyl-pipérazin-1-ium ;
- le chlorure de 1,4-bis-[(2-hydroxy-4-acétylamino-5-méthoxy-phénylcarbamoyl)-méthyl]-1-méthyl-pipérazin-1-ium ;
- le chlorure de 1,4-bis-[(2-hydroxy-6-amino-phénylcarbamoyl)-méthyl]-1-méthyl-pipérazin-1-ium ;
- le chlorure de 1,4-bis-[(2-hydroxy-4,6-diamino-phénylcarbamoyl)-méthyl]-1-méthyl-pipérazin-1-ium ;
- le chlorure de 1,4-bis-[(2-hydroxy-4-acétylamino-6-amino-phénylcarbamoyl)-méthyl]-1-méthyl-pipérazin-1-ium ;
- le chlorure de 1,4-bis-[(2-hydroxy-3,5-dichloro-4-méthyl-phénylcarbamoyl)-méthyl]-1-méthyl-pipérazin-1-ium ;
- le chlorure de 1,4-bis-[(2-hydroxy-3,5-dichloro-4-amino-phénylcarbamoyl)-méthyl]-1-méthyl-pipérazin-1-ium ;
- le chlorure de 1,4-bis-[(2-hydroxy-3,5-dichloro-4-acétylamino-phénylcarbamoyl)-méthyl]-1-méthyl-pipérazin-1-ium ;
et leurs sels d'addition avec un acide.

7. Composés selon l'une quelconque des revendications précédentes, **caractérisés par le fait que** les sels d'addition avec un acide sont choisis parmi les chlorhydrates, les bromhydrates, les sulfates, les citrates, les succinates, les tartrates, les lactates et les acétates.

8. Utilisation des composés de formule (I) tels que définis à l'une quelconque des revendications 1 à 7, à titre de coupleur pour la teinture d'oxydation des fibres kératiniques.

9. Composition pour la teinture d'oxydation des fibres kératiniques, **caractérisée par le fait qu'**elle contient, dans un milieu approprié pour la teinture :
- au moins une base d'oxydation, et
- au moins un coupleur choisi parmi les composés de formule (I) tels que définis à l'une quelconque des revendications 1 à 7.

10. Composition selon la revendication 9, **caractérisée par le fait que** le ou les composés de formule (I) et/ou le ou leurs sels d'addition avec un acide représentent de 0,0005 à 12 % en poids du poids total de la composition tinctoriale.

11. Composition selon la revendication 9 ou 10, **caractérisée par le fait que** la ou les bases d'oxydation sont choisies parmi les paraphénylènediamines, les bis-phénylalkylènediamines, les para-aminophénols, les ortho-aminophénols et les bases hétérocycliques, et leurs sels d'addition avec un acide.

12. Composition selon l'une quelconque des revendications 9 à 11, **caractérisée par le fait que** la ou les bases d'oxydation représentent de 0,0005 à 12 % en poids du poids total de la composition tinctoriale.

13. Composition selon l'une quelconque des revendications 9 à 12, **caractérisée par le fait qu'**elle renferme en plus du ou des composés de formule (I) ci-dessus, un ou plusieurs coupleurs additionnels choisis parmi les métaphénylènediamines, les méta-aminophénols, les métadiphénols et les coupleurs hétérocycliques, et leurs sels d'addition avec un acide, et/ou un ou plusieurs colorants directs.

14. Composition selon la revendication 13, **caractérisée par le fait que** le ou les coupleurs additionnels représentent de 0,0001 à 10 % en poids du poids total de la composition tinctoriale.

15. Composition selon l'une quelconque des revendications 9 à 14, **caractérisée par le fait que** les sels d'addition avec un acide sont choisis parmi les chlorhydrates, les bromhydrates, les sulfates, les citrates, les succinates, les tartrates, les lactates et les acétates.

16. Procédé de teinture d'oxydation des fibres kératiniques, **caractérisé par le fait que** l'on applique sur ces fibres au moins une composition tinctoriale telle que définie à l'une quelconque des revendications 9 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, **caractérisé par le fait que** l'agent oxydant est choisi parmi le peroxyde d'hydrogène, le peroxyde d'urée, les bromates de métaux alcalins, les persels, et les enzymes.

18. Dispositif à plusieurs compartiments, ou "kit" de teinture à plusieurs compartiments, dont un premier compartiment renferme une composition tinctoriale telle que définie à l'une quelconque des revendications 9 à 15 et un second compartiment renferme une composition oxydante.

## Patentansprüche

1. Kationische 2-Acylaminophenole der folgenden Formel (I) und deren Additionssalze mit einer Säure: 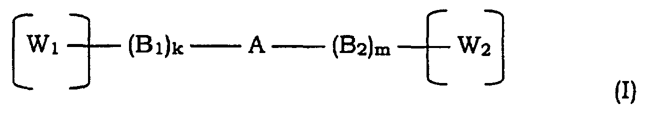 worin bedeuten:
· die Gruppe A Imidazolidinium, N-Alkyl(C₁₋₄)pyridinium, N-(2-Hydroxyethyl)pyridinium, Pyridinium, Dialkyl(C₁₋₄)ammonium, 1,4-Dimethylpiperazinium-1-yl;
· die Gruppen B₁ und B₂ unabhängig voneinander-(CH₂)-, -(CH₂)-(CH₂)- oder -(CH₂)-(CH₂)-(CH₂)-;
· k und m ganze Zahlen, die unabhängig voneinander die Werte 0 oder 1 annehmen könnten;
· W ₁ und W₂ unabhängig voneinander eine Gruppe W der folgenden Formel (II): 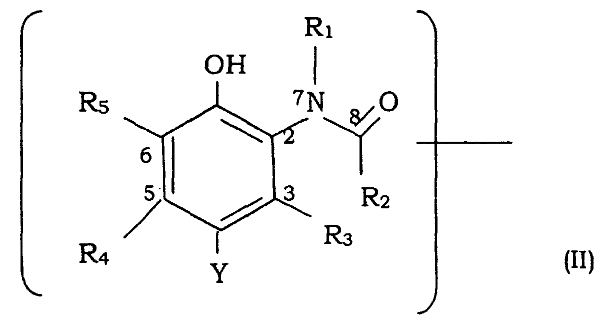 wobei in der Formel (II) bedeuten:
· R₁ Wasserstoff, Methyl, Ethyl, Isopropyl;
· R₂ eine Gruppe, die unter den folgenden Gruppen ausgewählt ist: Methyl, Ethyl, Propyl, Isopropyl, Butyl, Isobutyl, t-Butyl, Pentyl, Isopentyl, Neopentyl, Hexyl; Cyclopropyl, Cyclobutyl, Cyclopentyl, Cyclopentylmethyl, 3-Cyclopentylpropyl, Cyclohexyl, 2-Cyclohexylethyl, Hydroxymethyl, Methoxymethyl, 1,2-Dihydroxyethyl, 2-Carboxyethyl, 2-(Methoxycarbonyl)ethyl, 2-Carboxycyclopropyl, Methoxy, Ethoxy, Propoxy, Isopropoxy, Butoxy, Isobutoxy, Pentoxy, Neopentoxy, Hexyloxy, Cyclopentyloxy, Cyclohexyloxy, Amino, Methylamino, Ethylamino, Propylamino, Isopropylamino, Butylamino oder Cyclohexylamino;
· R₃ Wasserstoff, Chlor; Methyl, Hydroxymethyl, Methoxymethyl, 1-Hydroxyethyl, Aminomethyl, Methylaminomethyl, Hydroxy, Methoxy, Amino, Methylamino oder 2-Hydroxyethylamino;
· R₄ Wasserstoff, Chlor; Methyl, Ethyl, Hydroxymethyl, Methoxymethyl, Aminomethyl, Methylaminomethyl, Hydroxy, Methoxy, Acetoxy, Amino, Methylamino;
· R₅ Wasserstoff, Chlor, Brom; Methyl, Methoxy oder Methylamino;
· Y Wasserstoff, Chlor, Fluor oder Brom; Methoxy, Ethoxy, Propoxy, Benzyloxy, Phenoxy; -OCH₂CH₂OCH₃; -OCH₂CH₂OCH₃; -OCH₂CH₂N(CH₃)₂; -OCH₂(CO)OH, -OCH₂(CO)OCH₃, -OCH₂(CO)OC₂H₅;
mit der Maßgabe, dass:
· die Gruppe B₁ über ein beliebiges Atom der Gruppe A an die Gruppe A gebunden ist;
· wenn k = 0, die Gruppe A direkt über das Stickstoffatom der quartären Ammoniumgruppe an die Gruppe W₁ gebunden sein kann;
· die Gruppe B₂ über ein beliebiges Atom der Gruppe A an die Gruppe A gebunden ist;
· wenn m = 0, die Gruppe A direkt über das Stickstoffatom der quartären Ammoniumgruppe an die Gruppe W₂ gebunden sein kann;
· die Gruppe B₁ der Formel (I) an ein Atom der Gruppe W₁ gebunden ist und dieses Atom an dem Grundgerüst von W₁, das durch die nachstehend definierte Formel (III) dargestellt werden kann, mit einem Sternchen (*) gekennzeichnet ist;
· die Gruppe B₂ an ein Atom der Gruppe W₂ gebunden ist und dieses Atom an dem Grundgerüst von W₂, das durch die folgende Formel (III) dargestellt werden kann, mit einem Sternchen (*) gekennzeichnet ist:

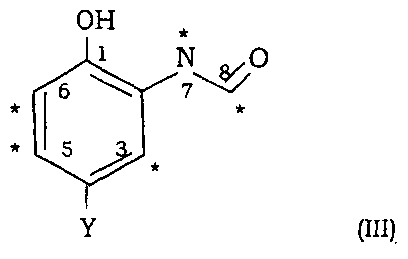

2. Verbindungen nach Anspruch 1, **dadurch gekennzeichnet, dass** R₂ eine Gruppe bedeutet, die unter Methyl, Ethyl, Propyl, 1,2-Dihydroxyethyl, 2-Carboxyethyl, Methoxy, Ethoxy, Propoxy, Amino, Ethylamino und Dimethylamino ausgewählt ist.

3. Verbindungen nach Anspruch 2, **dadurch gekennzeichnet, dass** R₂ Methyl, Methoxymethyl, 2-Carboxyethyl, Methoxy, Amino oder Ethylamino bedeutet.

4. Verbindungen nach Anspruch 1, **dadurch gekennzeichnet, dass** R₃ Wasserstoff; Methyl, Hydroxymethyl, Aminomethyl, Hydroxy, Methoxy, Amino oder Methylamino bedeutet.

5. Verbindungen nach Anspruch 1, **dadurch gekennzeichnet, dass** R₅ vorzugsweise Wasserstoff, Chlor, Methyl, Methoxy oder Methylamino bedeutet.

6. Verbindungen nach einem der vorhergehenden Ansprüche, **dadurch gekennzeichnet, dass** sie unter den folgenden Verbindungen ausgewählt sind:
■ 1,3-Bis[(2-hydroxy-phenylcarbamoyl)-methyl]-3H-imidazol-1-iumchlorid;
■ 1,3-Bis[(2-hydroxy-4-methyl-phenylcarbamoyl)-methyl]-3Himidazol-1-iumchlorid;
■ 1,3-Bis[(2-hydroxy-4-amino-phenylcarbamoyl)-methyl]-3Himidazol-1-iumchlorid;
■ 1,3-Bis[(2-hydroxy-4-acetylamino-phenylcarbamoyl)-methyl]-3H-imidazol-1-iumchlorid;
■ 1,3-Bis[(2-hydroxy-4-methoxycarbonylaminophenylcarbamoyl)-methyl]-3H-imidazol-1-iumchlorid;
■ 1,3-Bis[(2-hydroxy-5-chlor-phenylcarbamoyl)-methyl]-3Himidazol-1-iumchlorid;
■ 1,3-Bis[(2-hydroxy-4-methyl-5-chlor-phenylcarbamoyl)-methyl]-3H-imidazol-1-iumchlorid;
■ 1,3-Bis[(2-hydroxy-4-amino-5-chlor-phenylcarbamoyl]-methyl]-3H-imidazol-1-iumchlorid;
■ 1,3-Bis[(2-hydroxy-4-acetylamino-5-chlor-phenylcarbamoyl)-methyl]-3H-imidazol-1-iumchlorid;
■ 1,3-Bis[(2-hydroxy-5-methoxy-phenylcarbamoyl)-methyl]-3Himidazol-1-iumchlorid;
■ 1,3-Bis[(2-hydroxy-4-methyl-5-methoxy-phenylcarbamoyl)-methyl]-3H-imidazol-1-iumchlorid;
■ 1,3-Bis[(2-hydroxy-4-amino-5-methoxy-phenylcarbamoyl)-methyl]-3H-imidazol-1-iumchlorid;
■ 1,3-Bis[(2-hydroxy-4-acetylamino-5-methoxyphenylcarbamoyl)-methyl]-3H-imidazol-1-iumchlorid;
■ 1,3-Bis[(2-hydroxy-6-amino-phenylcarbamoyl)-methyl]-3Himidazol-1-iumchlorid;
■ 1,3-Bis[(2-hydroxy-4,6-diamino-phenylcarbamoyl)-methyl]-3Himidazol-1-iumchlorid;
■ 1,3-Bis[(2-hydroxy-4-acetylamino-6-amino-phenylcarbamoyl)-methyl]-3H-imidazol-1-iumchlorid;
■ 1,3-Bis[(2-hydroxy-3,5-dichlor-4-methyl-phenylcarbamoyl)-methyl]-3H-imidazol-1-iumchlorid;
■ 1,3-Bis[(2-hydroxy-3,5-dichlor-4-amino-phenylcarbamoyl)-methyl]-3H-imidazol-1-iumchlorid;
■ 1,3-Bis[(2-hydroxy-3,5-dichlor-4-acetylaminophenylcarbamoyl)-methyl]-3H-imidazol-1-iumchlorid;
■ 3-[(2-Hydroxy-4-amino-phenylcarbamoyl)-methyl]-1-[(2-hydroxy-4-methyl-phenylcarbamoyl)-methyl]-3H-imidazol-1-iumchlorid;
■ 3-[(2-Hydroxy-3,5-dichlor-4-methyl-phenylcarbamoyl)-methyl]-1-[(2-hydroxy-4-methyl-phenylcarbamoyl)-methyl]-3Himidazol-1-iumchlorid;
■ 1,4-Bis[(2-hydroxy-phenylcarbamoyl)-methyl]-1-methylpiperazin-1-iumchlorid;
■ 1,4-Bis[(2-hydroxy-4-methyl-phenylcarbamoyl)-methyl]-1-methyl-piperazin-1-iumchlorid;
■ 1,4-Bis[(2-hydroxy-4-amino-phenylcarbamoyl)-methyl]-1-methyl-piperazin-1-iumchlorid;
■ 1,4-Bis[(2-hydroxy-4-acetylamino-phenylcarbamoyl)-methyl]-1-methyl-piperazin-1-iumchlorid;
■ 1,4-Bis[(2-hydroxy-5-chlor-phenylcarbamoyl)-methyl]-1-methyl-piperazin-1-iumchlorid;
■ 1,4-Bis[(2-hydroxy-4-methyl-5-chlor-phenylcarbamoyl)-methyl]-1-methyl-piperazin-1-iumchlorid;
■ 1,4-Bis[(2-hydroxy-4-amino-5-chlor-phenylcarbamoyl)-methyl]-1-methyl-piperazin-1-iumchlorid;
■ 1,4-Bis[(2-hydroxy-4-acetylamino-5-chlor-phenylcarbamoyl)-methyl]-1-methyl-piperazin-1-iumchlorid;
■ 1,4-Bis[(2-hydroxy-5-methoxy-phenylcarbamoyl)-methyl]-1-methyl-piperazin-1-iumchlorid;
■ 1,4-Bis[(2-hydroxy-4-methyl-5-methoxy-phenylcarbamoyl)-methyl]-1-methyl-piperazin-1-iumchlorid;
■ 1,4-Bis[(2-hydroxy-4-amino-5-methoxy-phenylcarbamoyl)-methyl]-1-methyl-piperazin-1-iumchlorid;
■ 1,4-Bis[(2-hydroxy-4-acetylamino-5-methoxyphenylcarbamoyl)-methyl]-1-methyl-piperazin-1-iumchlorid;
■ 1,4-Bis[(2-hydroxy-6-amino-phenylcarbamoyl)-methyl]-1-methyl-piperazin-1-iumchlorid;
■ 1,4-Bis[(2-hydroxy-4,6-diamino-phenylcarbamoyl)-methyl]-1-methyl-piperazin-1-iumchlorid;
■ 1,4-Bis[(2-hydroxy-4-acetylamino-6-amino-phenylcarbamoyl)-methyl]-1-methyl-piperazin-1-iumchlorid;
■ 1,4-Bis[(2-hydroxy-3,5-dichlor-4-methyl-phenylcarbamoyl)-methyl]-1-methyl-piperazin-1-iumchlorid;
■ 1,4-Bis[(2-hydroxy-3,5-dichlor-4-amino-phenylcarbamoyl)-methyl]-1-methyl-piperazin-1-iumchlorid;
■ 1,4-Bis[(2-hydroxy-3,5-dichlor-4-acetylaminophenylcarbamoyl)-methyl]-1-methyl-piperazin-1-iumchlorid;
■ und deren Additionssalze mit einer Säure.

7. Verbindungen nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

8. Verwendung von Verbindungen der Formel (I) nach einem der Ansprüche 1 bis 7 als Kuppler zum oxidativen Färben von Keratinfasern.

9. Zusammensetzung zum oxidativen Färben von Keratinfasern, **dadurch gekennzeichnet, dass** sie in einem zum Färben geeigneten Medium enthält:
- mindestens eine Oxidationsbase, und
- mindestens einen Kuppler, der unten den Verbindungen der Formel (I) nach einem der Ansprüche 1 bis 7 ausgewählt ist.

10. Zusammensetzung nach Anspruch 9, **dadurch gekennzeichnet, dass** die Verbindung(en) der Formel (I) und/ oder ihr(e) Additionssalz(e) mit einer Säure 0,0005 bis 12 Gew.-% des Gesamtgewichts der Farbmittelzusammensetzung ausmachen.

11. Zusammensetzung nach Anspruch 9 oder 10, **dadurch gekennzeichnet, dass** die die Oxidationsbase(n) unter den p-Phenylendiaminen, Bisphenylalkylendiaminen, p-Aminophenolen, o-Aminophenolen und den heterocyclischen Basen und ihren Additionssalzen mit einer Säure ausgewählt sind.

12. Zusammensetzung nach einem der Ansprüche 9 bis 11, **dadurch gekennzeichnet, dass** die Oxidationsbase(n) 0,0005 bis 12 Gew.-% des Gesamtgewichts der Farbmittelzusammensetzung ausmachen.

13. Zusammensetzung nach einem der Ansprüche 9 bis 12, **dadurch gekennzeichnet, dass** sie neben der oder den Verbindungen der Formel (I) ferner einen oder mehrere zusätzliche Kuppler, die unter den m-Phenylendiaminen, m-Aminophenolen, m-Dihydroxybenzolen und den heterocyclischen Kupplern ausgewählt sind, und deren Additionssalze mit einer Säure und/ oder einen oder mehrere Direktfarbstoffe enthält.

14. Zusammensetzung nach Anspruch 13, **dadurch gekennzeichnet, dass** der oder die zusätzlichen Kuppler 0,0001 bis 10 Gew.-% des Gesamtgewichts der Farbmittelzusammensetzung ausmachen.

15. Zusammensetzung nach einem der Ansprüche 9 bis 14, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

16. Verfahren zum oxidativen Färben von Keratinfasern, **dadurch gekennzeichnet, dass** auf die Fasern mindestens eine Farbmittelzusammensetzung nach einem der Ansprüche 9 bis 15 aufgetragen wird und die Farbe bei einem sauren, neutralen oder alkalischen pH-Wert mit einem Oxidationsmittel entwickelt wird, das kurz vor der Anwendung zu der Farbmittelzusammensetzung gegeben wird oder das in einer oxidierenden Zusammensetzung vorliegt, die gleichzeitig oder getrennt davon anschließend aufgetragen wird.

17. Verfahren nach Anspruch 16, **dadurch gekennzeichnet, dass** das Oxidationsmittel unter Wasserstoffperoxid, Harnstoffperoxid, Alkalimetallbromaten, Salzen von Persäuren und Enzymen ausgewählt ist.

18. Vorrichtung mit mehreren Abteilungen oder "Kit" zum Färben mit mehreren Abteilungen, wobei eine'erste Abteilung eine Farbmittelzusammensetzung nach einem der Ansprüche 9 bis 15 und eine zweite Abteilung eine oxidierende Zusammensetzung enthält.

## Claims

1. Cationic 2-acylaminophenols of formula (I) below, and the addition salts thereof with an acid: 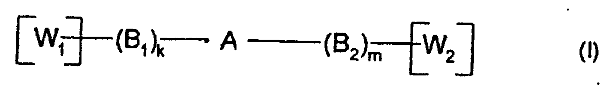 in which:
- the group A represents an imidazolidinium, N-(C₁-C₄)alkylpyridinium, N-(2-hydroxyethyl)pyridinium, pyridinium, di(C₁-C₄)alkylammonium or 1,4-dimethylpiperazinium-1-yl group;
- B₁ and B₂, independently of each other, represent a -(CH₂)-, -(CH₂)-(CH₂)- or -(CH₂)-(CH₂)-(CH₂)- arm;
- k and m are integers which can take, independently of each other, the value 0 or 1;
- W₁ and W₂ represent, independently of each other, a group W represented by formula (II) below: 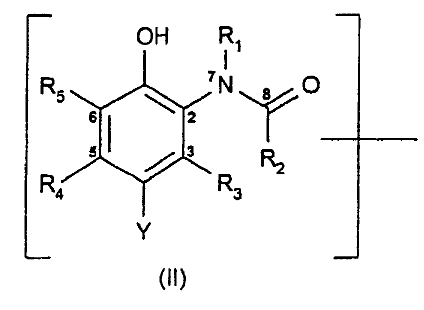 in which formula (II):
• R₁ represents a hydrogen atom; or a methyl, ethyl or isopropyl radical;
• R₂ represents a radical chosen from methyl, ethyl, propyl, isopropyl, butyl, isobutyl, tert-butyl, pentyl, isopentyl, neopentyl, hexyl; cyclopropyl, cyclobutyl, cyclopentyl, cyclopentylmethyl, 3-cyclopentylpropyl, cyclohexyl, 2-cyclohexylethyl, hydroxymethyl, methoxymethyl, 1,2-dihydroxyethyl, 2-carboxyethyl, 2-(methoxycarbonyl)ethyl, 2-carboxycyclopropyl; methoxy, ethoxy, propoxy, isopropoxy, butoxy, isobutoxy, pentoxy, neopentoxy, hexyloxy, cyclopentyloxy, cyclohexyloxy; amino, methylamino, ethylamino, propylamino, isopropylamino, butylamino, cyclohexylamino and dimethylamino;
• R₃ represents a hydrogen or chlorine atom; or a methyl, hydroxymethyl, methoxymethyl, 1-hydroxyethyl, aminomethyl, methylaminomethyl, hydroxyl, methoxy, amino, methylamino or 2-hydroxyethylamino radical;
• R₄ represents a hydrogen or chlorine atom; or a methyl, ethyl, hydroxymethyl, methoxymethyl, aminomethyl, methylaminomethyl, hydroxyl, methoxy, acetoxy, amino or methylamino radical;
• R₅ represents a hydrogen, chlorine or bromine atom; or a methyl, methoxy or methylamino radical;
• Y represents a hydrogen, chlorine, fluorine or bromine atom; a methoxy, ethoxy, propoxy, benzyloxy or phenoxy group; or an -OCH₂CH₂OCH₃, -OCH₂CH₂OCH₃, -OCH₂CH₂N(CH₃)₂ , -OCH₂(CO)OH, -OCH₂(CO)OCH₃ or -OCH₂(CO)OC₂H₅ group;
it being understood that:
- the radical B₁ is linked to the group A via any one of the atoms in the radical A;
- when k = 0, then the group A can be linked to the group W₁ directly via the nitrogen atom of the quaternary ammonium;
- the radical B₂ is linked to the group A via any one of the atoms in the radical A;
- when m = 0, then the group A. can be linked to the group W₂ directly via the nitrogen atom of the quaternary ammonium;
- the group B₁ of formula (I) is linked to an atom of the group W₁ and this atom is identified by an asterisk (*) on the skeleton of W₁ represented by formula (III) defined below;
- the group B₂ is linked to an atom of the group W₂ and this atom is identified by an asterisk (*) on the skeleton of W₂ represented by formula (III) defined below;

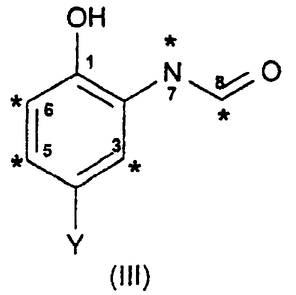

2. Compounds according to Claim 1, **characterized in that** R₂ represents a radical chosen from a methyl, ethyl, propyl, 1,2-dihydroxyethyl, 2-carboxyethyl, methoxy, ethoxy, propoxy, amino, ethylamino and dimethylamino radical.

3. Compounds according to Claim 2, **characterized in that** R₂ represents a methyl, methoxymethyl, 2-carboxyethyl, methoxy, amino or ethylamino radical.

4. Compounds according to Claim 1, **characterized in that** R₃ represents a hydrogen atom or a methyl, hydroxymethyl, aminomethyl, hydroxyl, methoxy, amino or methylamino radical.

5. Compounds according to Claim 1, **characterized in that** R₅ represents a hydrogen or chlorine atom or a methyl, methoxy or methylamino radical.

6. Compounds according to any one of the preceding claims, **characterized in that** they are chosen from:
- 1,3-bis[(2-hydroxyphenylcarbamoyl)methyl]-3H-imidazol-1-ium chloride;
- 1,3-bis[(2-hydroxy-4-methylphenylcarbamoyl)methyl]-3H-imidazol-1-ium chloride;
- 1,3-bis[(2-hydroxy-4-aminophenylcarbamoyl)methyl]-3H-imidazol-1-ium chloride;
- 1,3-bis[(2-hydroxy-4-acetylaminophenylcarbamoyl)methyl]-3H-imidazol-1-ium chloride;
- 1,3-bis[(2-hydroxy-4-methoxycarbonylaminophenylcarbamoyl)methyl]-3H-imidazol-1-ium chloride;
- 1,3-bis[(2-hydroxy-5-chlorophenylcarbamoyl)methyl]-3H-imidazol-1-ium chloride;
- 1,3-bis[(2-hydroxy-4-methyl-5-chlorophenylcarbamoyl)methyl]-3H-imidazol-1-ium chloride;
- 1,3-bis[(2-hydroxy-4-amino-5-chlorophenylcarbamoyl)methyl]-3H-imidazol-1-ium chloride;
- 1,3-bis[(2-hydroxy-4-acetylamino-5-chlorophenylcarbamoyl)methyl]-3H-imidazol-1-ium chloride;
- 1,3-bis[(2-hydroxy-5-methoxyphenylcarbamoyl)methyl]-3H-imidazol-1-ium chloride;
- 1,3-bis[(2-hydroxy-4-methyl-5-methoxyphenylcarbamoyl)methyl]-3H-imidazol-1-ium chloride;
- 1,3-bis[(2-hydroxy-4-amino-5-methoxyphenylcarbamoyl)methyl]-3H-imidazol-1-ium chloride;
- 1,3-bis[(2-hydroxy-4-acetylamino-5-methoxyphenylcarbamoyl)methyl]-3H-imidazol-1-ium chloride;
- 1,3-bis[(2-hydroxy-6-aminophenylcarbamoyl)methyl]-3H-imidazol-1-ium chloride;
- 1,3-bis[(2-hydroxy-4,6-diaminophenylcarbamoyl)methyl]-3H-imidazol-1-ium chloride;
- 1,3-bis[(2-hydroxy-4-acetylamino-6-aminophenylcarbamoyl)methyl]-3H-imidazol-1-ium chloride;
- 1,3-bis[(2-hydroxy-3,5-dichloro-4-methylphenylcarbamoyl)methyl]-3H-imidazol-1-ium chloride;
- 1,3-bis[(2-hydroxy-3,5-dichloro-4-aminophenylcarbamoyl)methyl]-3H-imidazol-1-ium chloride;
- 1,3-bis[(2-hydroxy-3,5-dichloro-4-acetylaminophenylcarbamoyl)methyl]-3H-imidazol-1-ium chloride;
- 3-[(2-hydroxy-4-aminophenylcarbamoyl)methyl]-1-[(2-hydroxy-4-methylphenylcarbamoyl)methyl]-3H-imidazol-1-ium chloride;
- 3-[(2-hydroxy-3,5-dichloro-4-methylphenylcarbamoyl)methyl]-1-[(2-hydroxy-4-methylphenylcarbamoyl)methyl]-3H-imidazol-1-ium chloride;
- 1,4-bis[(2-hydroxyphenylcarbamoyl)methyl]-1-methylpiperazin-1-ium chloride;
- 1,4-bis[(2-hydroxy-4-methylphenylcarbamoyl)methyl]-1-methylpiperazin-1-ium chloride;
- 1,4-bis[(2-hydroxy-4-aminophenylcarbamoyl)methyl]-1-methylpiperazin-1-ium chloride;
- 1,4-bis[(2-hydroxy-4-acetylaminophenylcarbamoyl)methyl]-1-methylpiperazin-1-ium chloride;
- 1,4-bis[(2-hydroxy-5-chlorophenylcarbamoyl)methyl]-1-methylpiperazin-1-ium chloride;
- 1,4-bis[(2-hydroxy-4-methyl-5-chlorophenylcarbamoyl)methyl]-1-methylpiperazin-1-ium chloride;
- 1,4-bis[(2-hydroxy-4-amino-5-chlorophenylcarbamoyl)methyl]-1-methylpiperazin-1-ium chloride;
- 1,4-bis[(2-hydroxy-4-acetylamino-5-chlorophenylcarbamoyl)methyl]-1-methylpiperazin-1-ium chloride;
- 1,4-bis[(2-hydroxy-5-methoxyphenylcarbamoyl)methyl]-1-methylpiperazin-1-ium chloride;
- 1,4-bis[(2-hydroxy-4-methyl-5-methoxyphenylcarbamoyl)methyl]-1-methylpiperazin-1-ium chloride;
- 1,4-bis[(2-hydroxy-4-amino-5-methoxyphenylcarbamoyl)methyl]-1-methylpiperazin-1-ium chloride;
- 1,4-bis[(2-hydroxy-4-acetylamino-5-methoxyphenylcarbamoyl)methyl]-1-methylpiperazin-1-ium chloride;
- 1,4-bis[(2-hydroxy-6-aminophenylcarbamoyl)methyl]-1-methylpiperazin-1-ium chloride;
- 1,4-bis[(2-hydroxy-4,6-diaminophenylcarbamoyl)methyl]-1-methylpiperazin-1-ium chloride;
- 1,4-bis[(2-hydroxy-4-acetylamino-6-aminophenylcarbamoyl)methyl]-1-methylpiperazin-1-ium chloride;
- 1,4-bis[(2-hydroxy-3,5-dichloro-4-methylphenylcarbamoyl)methyl]-1-methylpiperazin-1-ium chloride;
- 1,4-bis[(2-hydroxy-3,5-dichloro-4-aminophenylcarbamoyl)methyl]-1-methylpiperazin-1-ium chloride;
- 1,4-bis[(2-hydroxy-3,5-dichloro-4-acetylaminophenylcarbamoyl)methyl]-1-methylpiperazin-1-ium chloride;
and the addition salts thereof with an acid.

7. Compounds according to any one of the preceding claims, **characterized in that** the addition salts with an acid are chosen from the hydrochlorides, hydrobromides, sulphates, citrates, succinates, tartrates, lactates and acetates.

8. Use of the compounds of formula (I) as defined in any one of Claims 1 to 7, as couplers for the oxidation dyeing of keratin fibres.

9. Composition for the oxidation dyeing of keratin fibres, **characterized in that** it contains, in a medium which is suitable for dyeing:
- at least one oxidation base, and
- at least one coupler chosen from the compounds of formula (I) as defined in any one of Claims 1 to 7.

10. Composition according to Claim 9, **characterized in that** the compound(s) of formula (I) and/or the addition salt(s) thereof with an acid represent(s) from 0.0005% to 12% by weight relative to the total weight of the dye composition.

11. Composition according to Claim 9 or 10, **characterized in that** the oxidation base(s) is(are) chosen from para-phenylenediamines, bis(phenyl)alkylenediamines, para-aminophenols, ortho-aminophenols and heterocyclic bases, and the addition salts thereof with an acid.

12. Composition according to any one of Claims 9 to 11, **characterized in that** the oxidation base(s) represent(s) from 0.0005% to 12% by weight relative to the total weight of the dye composition.

13. Composition according to any one of Claims 9 to 12, **characterized in that**, in addition to the compound(s) of formula (I) above, it contains one or more additional couplers chosen from meta-phenylenediamines, meta-aminophenols, meta-diphenols and heterocyclic couplers, and the addition salts thereof with an acid, and/or one or more direct dyes.

14. Composition according to Claim 13, **characterized in that** the additional coupler(s) represent(s) from 0.0001% to 10% by weight relative to the total weight of the dye composition.

15. Composition according to any one of Claims 9 to 14, **characterized in that** the addition salts with an acid are chosen from the hydrochlorides, hydrobromides, sulphates, citrates, succinates, tartrates, lactates and acetates.

16. Process for the oxidation dyeing of keratin fibres, **characterized in that** at least one dye composition as defined in any one of Claims 9 to 15 is applied to these fibres, and **in that** the colour is developed at acidic, neutral or alkaline pH using an oxidizing agent which is added to the dye composition just at the time of use, or which is present in an oxidizing composition applied simultaneously or sequentially in a separate manner.

17. Process according to Claim 16, **characterized in that** the oxidizing agent is chosen from hydrogen peroxide, urea peroxide, alkali metal bromates, persalts and enzymes.

18. Multi-compartment device or multi-compartment dyeing kit, a first compartment of which contains a dye composition as defined in any one of Claims 9 to 15 and a second compartment of which contains an oxidizing composition.
